(19) Europäisches Patentamt
European Patent Office
Office européen des brevets

(11) Numéro de publication: **0 149 581**
**B1**

(12) # FASCICULE DE BREVET EUROPÉEN

(45) Date de publication du fascicule du brevet: **22.07.87**

(21) Numéro de dépôt: **85400080.9**

(22) Date de dépôt: **17.01.85**

(51) Int. Cl.[4]: **C 12 P 21/00,** A 61 K 39/395, A 61 K 39/44 // A61K39/39

(54) **Nouvelles compositions contenant des oligo-muramylpeptides et leur application, notamment pour l'activation des macrophages.**

(30) Priorité: **17.01.84 FR 8400676**

(43) Date de publication de la demande: **24.07.85 Bulletin 85/30**

(45) Mention de la délivrance du brevet: **22.07.87 Bulletin 87/30**

(84) Etats contractants désignés: **AT BE CH DE GB IT LI LU NL SE**

(56) Documents cité:
**EP-A-0 003 833**
**EP-A-0 089 290**

**CHEMICAL ABSTRACTS, vol. 102, no. 13, 1 avril 1985, no. 111100b, Columbus, Ohio, US; A.C. ROCHE et al.: "Selective macrophage activation by muramyldipeptide bound to monoclonal antibodies specific for mouse tumor cells"**
**BIOLOGICAL ABSTRACTS, vol. 78, 1984, no. 77290, Philadelphia, US; C. LECLERC et al.: "Marked enhancement of macrophage activation induced by synthetic muramyl dipeptide conjugate using monoclonal anti-muramyl dipeptide antibodies"**
**BIOLOGICAL ABSTRACTS, vol. 77, no. 9, 1984, no. 67541, Philadelphia, US; G.M. BAHR et al.: "Monoclonal antibodies to the synthetic adjuvant muramyl dipeptide. Characterization of the specificity"**

(73) Titulaire: **ANVAR Agence Nationale de Valorisation de la Recherche, 43, rue Caumartin, F-75436 Paris Cédex 09 (FR)**

(72) Inventeur: **Leclerc, Claude, 127, rue de Javel, F-75015 Paris (FR)**
Inventeur: **Bahr, Georges, 84, boulevard Pasteur, F-75015 Paris (FR)**
Inventeur: **Chedid, Louis, 16 - 18, Rue Gaston de Caillavet, F-75015 Paris (FR)**

(74) Mandataire: **Gutmann, Ernest, S.C. Ernest Gutmann - Yves Plasseraud 67, boulevard Haussmann, F-75008 Paris (FR)**

**EP 0 149 581 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

L'invention est relative à de nouvelles compositions contenant des muramylpeptides et possédant d'importantes propriétés d'activation des macrophages. Elle concerne également des compositions pour réaliser l'activation des macrophages in vivo, ces compositions contenant ces nouveaux dérivés de muramylpeptides en association avec un véhicule biologiquement acceptable, en vue de l'administration à un hôte vivant.

On désigne en général sous l'expression "muramylpeptides" les produits comportant un motif saccharidique, notamment du type N-acétyl-muramyle ou N-acétyl-normuramyle, et une chaîne peptidique rattachée à ce motif saccharidique. Le premier résidu aminoacyle de cette chaîne peptidique (et rattaché au motif saccharidique) peut correspondre à l'un quelconque des aminoacides naturels (le cas échéant aussi aux isomères dextrogyres correspondants), voire même à des dérivés de substitution de ces aminoacides. Le second résidu aminoacyle est en général dérivé de l'acide glutamique. Les fonctions carboxyle de ce dernier peuvent être engagées dans des liaisons chimiques, notamment ester ou amide ou encore, notamment en ce qui concerne le groupe gamma-carboxyle, avec un autre résidu aminoacyle. Des résidus aminoacyle supplémentaires préférés sont constitués par des groupes L-lysyle ou alanyle, notamment L-lysyle ou L-alanyle.

De nombreuses études ont été réalisées sur le MDP (N-acétyl-muramyl-L-alanyl-D-isoglutamine), en tant qu'éventuel agent susceptible de stimuler l'immunité faisant intervenir les macrophages et plus particulièrement une immunité antitumorals. Le MDP lui-même, dont est bien connue l'activité stimulants non spécifique de la réponse immunitaire à l'égard d'antigènes déterminés, témoigne cependant d'une activité réduite à l'égard de l'immunité à médiation cellulaire. Pour renforcer l'immunité à médiation cellulaire du MDP ou d'autres muramylpeptides, diverses transformations de ces molécules ont été envisagées. Parmi ces transformations, on retiendra essentiellement les produits résultant de la conjugaison des groupes muramylpeptides à uns molécule porteuse, tels que des polymères peptidiques de synthèse et plus particulièrement des polymères de L-lysine portant des ramifications de résidus aminoacyle distincts, notamment D- ou L-alanyle ou les deux à la fois. On peut se reporter utilement au brevet français n° 7 900 819 (2 415 224) pour ce qui est de la constitution de telles molécules porteuses. On rappellers que, dans ce brevet, les polypeptides du genre en question jouaient le rôle de molécule porteuse à l'égard de muramylpeptides dans lesquels les premier et second résidus aminoacyle de la chaîne peptidique reliée au groupe muramyle étaient tous deux dextrogyres. Les produits résultant de la susdite conjugaison entre des molécules de MDP ou analogues et de telles molécules porteuses possédant en effet des propriétés de stimulation de l'immunité à médiation cellulaire in vivo. Ils présentent cependant également un certain niveau de toxicité, de sorte que les doses susceptibles d'être mises en oeuvre doivent être maintenues dans diverses limites.

Il a également été proposé de conjuguer des muramylpeptides à des antigènes, par l'intermédiaire de liaisons covalentes (brevet européen n° 0 003 833). Ces produits de conjugaison sont d'un intérêt particulier lorsqu'est recherché un accroissement sensible de la stimulation de la réponse immunitaire à l'égard de l'antigène entrant dans ce conjugué. L'utilisation effective de ces conjugués aux fins décrites dans le brevet n'est pas non plus sans inconvénient, dès lors que les doses à mettre en oeuvre deviennent relativement importantes.

L'invention a pour but de remédier au moins en partie à ces difficultés, et plus particulièrement de fournir de nouvelles compositions à base de muramylpeptide, caractérisées par une capacité accrue d'activation des macrophages, que cette activité soit non spécifique ou au contraire spécifique et orientée contre un antigène déterminé, et ce à des doses extrêmement faibles.

Les compositions selon l'invention sont constituées par l'association d'un muramylpeptide ou d'un oligo-muramylpeptide et d'au moins un anticorps apte à reconnaître ce muramylpeptide ou cet oligomuramylpeptide, en d'autres termes à former un complexe avec ce muramylpeptide ou cet oligomuramylpeptide, plus particulièrement un immun-complexe.

Les compositions selon l'invention présentent des capacités d'activation de macrophages à des doses remarquablement plus faibles que les dérivés de muramylpeptides de l'état de la technique. En particulier, elles possèdent des propriétés de stimulation de l'immunité à médiation cellulaire qui les rendent particulièrement utiles à plusieurs niveaux. Elles sont par elles-mêmes capables d'exercer une immunité renforcée à l'égard des proliférations de cellules tumorales. Lorsqu'elles sont administrées en association ou en combinaison avec un antigène déterminé, elles manifestent un effet d'adjuvant immunologique extrêmement efficace à des doses infinitésimales.

D'une façon générale, l'expression muramylpeptide, telle qu'elle sera utilisée ci-après désignera toute molécule comprenant un ou plusieurs motifs muramylpeptide dans sa molécule. Il s'agit par exemple d'un oligomère de muramylpeptide, ou encore du produit résultant de la fixation de groupes muramylpeptides sur une molécule porteuse, notamment un polypeptide synthétique du genre déjà rappelé plus haut, ou encore sur une protéine naturelle, telle qu'une sérum-albumine, une hémocyanine ou une anatoxine vaccinante, par exemple l'anatoxine tétanique. A ces muramylpeptides se rattachent encore ceux dans lesquels des groupes muramylpeptides sont fixés sur un antigène ou un haptène porteur d'un site ou déterminant antigénique, caractéristique de l'antigène, en ce que la conjugaison de l'haptène avec un muramylpeptide conduit à une molécule susceptible d'induire in vivo la production d'anticorps contre ledit antigène.

L'anticorps peut être un sérum polyclonal ou sa fraction immunoglobulinique, obtenue à partir d'un sérum

d'un animal préalablement immunisé contre le muramylpeptide concerné ou un muramylpeptide analogue. De préférence, l'anticorps considéré sera un anticorps monoclonal, et de préférence encore un anticorps monoclonal réagissant sélectivement contre la structure muramylpeptide globale, et ne réagissant que peu ou pas du tout contre soit la partie muramyle, soit la partie peptidique. Sous l'expression anticorps, on entendra encore tout fragment d'anticorps susceptible d'être reconnu par un récepteur porté par les membranes de macrophages.

D'une façon générale, que l'anticorps soit monoclonal ou polyclonal, ou encore fragmentaire il ést préférable que son site Fc reste disponible et fonctionnel, dans la mesure où l'anticorps est apte à se fixer par l'intermédiaire de ce site, sur le récepteur correspondant porté par la membrane du macrophaqe.

Il est à cet égard important de noter que l'invention s'étend aussi à des compositions dans lesquelles le muramylpeptide et l'anticorps (ou fragment d'anticorps) seraient couplés l'un à l'autre, notamment par l'intermédiaire d'une liaison covalente obtenue par des procédés classiques par exemple dans la synthèse des protéines. Mais des compositions préférées de l'invention sont celles dans lesquelles la liaison entre l'anticorps et le muramylpeptide est du type liaison immunologique antigène-anticorps. Comme il sera montré dans la description la liaison immunologique ne doit pas interférer avec le site de fixation de l'anticorps sur les macrophages. Ceci étant, la liaison immunologique est particulièrement préférée, parce qu'elle peut être réalisée de façon spontanée, par simple mise en contact du muramylpeptide et de l'anticorps dirigé contre lui, donc sans qu'il soit nécessaire d'avoir recours aux techniques, souvent compliquées, de couplage chimique entre le muramylpeptide et l'autre partie de la composition.

D'une façon générale, il va de soi que la liaison, covalente ou non, entre le muramylpeptide et l'anticorps devra toujours être telle qu'elle n'interfère pas avec le site actif de l'anticorps à l'égard du récepteur correspondant des macrophages. La vérification de l'absence d'interférence entre les sites présumés distincts qui, au niveau de l'anticorps, apparaissent comme intervenant dans ces deux types de réaction, pourra chaque fois être effectuée par un test d'activité biologique in vivo de la composition obtenue, précisément au niveau de l'activation des macrophages. On a en effet constaté que l'association avec l'anticorps se manifestait par une exaltation considérable de l'activité stimulante du muramylpeptide. Cette exaltation est particulièrement remarquable car l'activation considérable des macrophages pouvait a priori être considérée comme inattendue. On aurait en effet pu s'attendre à ce gue la présence de l'anticorps soit plutot de nature a inhiber l'activite stimulante du MDP.

Comme on l'a déjà remarqué, les compositions de l'invention sont actives à des doses excessivement faibles. Les résultats observés peuvent en effet être interprétés comme résultant d'un ciblage particulièrement efficace du muramylpeptide sur les macrophages. On peut même être tenté de formuler l'hypothèse que l'action de stimulation n'est due qu'à la faible proportion de muramylpeptide éventuellement internalisé à l'intérieur des macrophages, par l'intermédiaire de l'anticorps, éventuellement après séparation de ce dernier au niveau de la paroi cellulaire.

En ce qui concerne les molécules porteuses faisant éventuellement partie des muramylpeptides de l'invention, il sera toujours préférable d'utiliser des molécules qui, de toute façon ne conduiraient qu'à des réactions antigéniques faibles chez l'hôte, même lorsqu'elles sont administrées à fortes doses. En l'occurrence, dans le cas ou les molécules porteuses sont elles-mêmes d'origine biologique (cas des immunoglobulines par exemple), il est préférable d'avoir recours à des molécules originaires du même hôte que celui auquel les compositions selon l'invention sont destinées. Dans le cas de compositions destinées ä l'homme, la molécule porteuse sera égalemént avantageusement d'origine humaine. Les molécules porteuses ne peuvent alors conduire dans la pratique à aucune activité biologique réelle en raison des doses excessivement faibles mises en oeuvre, tant en valeur absolue que vis-à-vis des proportions importantes des molécules circulantes de l'hôte, par exemple des immunoglobulines circulantes.

Il n'y a pas de limitation è retenir à l'égard des muramylpeptides eux-mêmes dont le caractère généralement adjuvant découle de la littérature technique abondante disponible à ce jour. Pour mémoire, on fera néanmoins référence ci-après aux brevets et demandes de brevets dans lesquels des catégories de muramylpeptides susceptibles d'être mis en oeuvre également dans le cadre de la présente invention ont été définies. A titre non limitatif, on signalera les familles de muramylpeptides définies ou envisagées dans les brevets français n° 7 422 909 (2 292 486), 7 606 821 (2 343 484), 7 808 049 (2 420 545), 7 816 793 (2 428 051) et 7 900 819 (2 446 292), le brevet américain n° 4 153 684 et les demandes de brevet européen n° 0 003 833, 0 025 495 et 0 089 290.

Sans qu'il y ait lieu d'y voir une restriction à la portée de l'invention revendiquée, on observera que la catégorie préférée de muramylpeptides susceptibles d'être mis en oeuvre est celle répondant à la formule générale suivante:

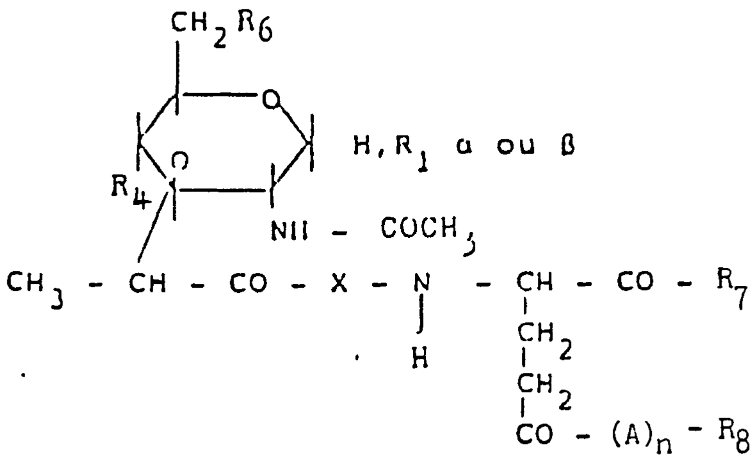

dans laquelle les substituants $R_1$, $R_4$, $R_6$, X, $R_7$, $R_8$, A et n ont respectivement les significationa suivantes:

- $R_1$ est $-NH_2$, -OH; -NY ou -OY, Y représentant un groupe substitué ou non par un groupe amino, ce groupe étant choisi parmi alcoyle, aryle ou alcoyl-aryle portant un maximum de 10 atomes de carbone;
- $R_4$ est hydroxyle, acyloxy ou alcoxy ayant au plus 4 atomes de carbone, ou monosuccinyle;
- $R_6$ est $-NH_2$, -OH,-NHZ ou -OZ, Z étant un radical linéaire ou ramifié formé par acyle ou alkyle contenant de 1 à 90 atomes de carbone et portant, le cas échéant, des substituanta hydroxyle, carboxyle, carbonyle, amino, cyclopropyle ou méthoxyle;
- X est un résidu aminoacyle du groupe comprenant:

alanyle, arginyle, asparagyle, aspartyle, cystéinyle, glutaminyle, glutamyle, glycyle, histidyle, hydroxypropyle, isoleucyle, leucyle, lysyle, méthionyle, phénylalanyle, propyle, séryle, thréonyle, tryptophanyle, tyrosyle et valyle, N-méthyl-alanyle;

- $R_7$ est un groupe -OH, $-NH_2$, alcoylamide, alcoxy linéaire ou ramifié, aryle ou alcoxy-aryle comprenant au plus 10 atomes de carbone,
- $R_8$ est un groupe -OH, $-NH_2$ ou un groupe alcoxy comprenant de 1 à 10 atomes de carbone,
- A représente un acide aminé tel qu'un résidu alanyle, lysine, glutamine et
- n est égal à 0,1 ou 2.

De préférence, les acides aminés intervenant dans la constitution de la chaîne peptidique sont (à l'exception de la glycine) lévogyres.

Des oligo-muramylpeptides préférés sont formés des polymères comportant une chaîne de nature peptidique soit naturelle, soit synthétique, sur laquelle sont greffés des motifs muramylpeptides, par l'intermédiaire de liaisons covalentes. Une catégorie préférée de polymères de ce type comporte une chaîne dont les maillons sont des résidus aminoacides correspondant au groupe des acides aminés comprenant l'acide alpha, bêta-diaminobutyrique, l'ornithine, la lysine, l'homo-lysine, les fonctions amines latérales pouvant porter les ramifications peptidiques dont les maillons correspondent aux résidus aminoacides de l'alanine, de la glycine, de l'acide alpha-aminobutyrique, de la valine, de la leucine, de la proline, le susdit polymère peptidique présentant une masse moléculaire moyenne comprise entre environ 50.000 et 250.000, et sur lequel sont liés de façon covalente des motifs muramylpeptide dont le premier résidu aminoacide (lié au groupe muramyle) est un stéréo-isomère de la série L, le second résidu aminoacide présentant la structure de l'acide D-glutamique.

Parmi ces polymères, celui dont la chaîne est une polylysine portant des ramifications peptidiques polyalanyle est préférée. D'autres caractéristiques préférées des chaînes en question (lesquelles jouent le rôle de molécules porteuses de l'oligo-muramylpeptide envisagé dans le cadre de la présente invention) résultent du brevet France n° 7 900 819. Cette catégorie d'oligo-muramylpeptides peut être préparée par des procédés en tous points semblables à ceux qui ont été décrits dans le brevet précité, si ce n'est que dans les produits de départ mis en oeuvre pour constituer le fragment peptidique de l'oligo-muramylpeptide, le résidu L-alanyle est substitué au résidu D-alanyle.

Une autre catégorie d'oligo-muramylpeptides avantageusement mis en oeuvre dans le cadre de la présente invention consiste en des oligomères de muramylpeptides du type de ceux qui ont été décrits dans le brevet France n° 7 816 792 (2 428 050).

Les proportions relatives de motifs muramylpeptide vis-à-vis de l'anticorps peuvent varier dans de grandes proportions, pour autant que soit maintenu le caractère de solubilité dans l'eau des complexes obtenus. A titre indicatif, on mentionnera que la proportion pondérale de motifs muramylpeptide vis-à-vis de la totalité de la molécule sera de préférence comprise entre 5 % et 50 %. On peut aussi mentionner que des complexes préférés conformes à l'invention peuvent comprendre de 5 à 30 motifs muramylpeptide par molécule-vecteur.

Les compositions selon l'invention peuvent être mises en oeuvre seules ou en association ou combinaison

avec un antigène déterminé. La composition selon l'invention sera utilisée seule dans les cas où une stimulation générale de l'activation des macrophages sera recherchée, notamment pour accroître la résistance de l'hôte contre des infections bactériennes ou virales, ou contre des proliférations de cellules tumorales. Par contre, lorsque l'on souhaitera bénéficier des propriétés stimulantes non spécifiques de l'immunité contre des antigènes déterminés, les compositions selon l'invention pourront être administrées à l'hôte, conjointement avec l'antigène. Le muramylpeptide ou oligomuramylpeptide, d'une part, l'anticorps, d'autre part, et l'antigène, d'autre part encore, peuvent être administrés simultanément ou séparément selon un protocole préalablement établi, pour obtenir le meilleur effet de synergie souhaité. On peut aussi avoir recours ä des compositions contenant des produits de combinaison entre le muramylpeptide et l'antigène particulier contre lequel une protection est recherchée, sous forme d'immun-complexe préparé d'avance ou non, avec l'anticorps.

Des caractéristiques supplémentaires de l'invention apparaîtront encore au cours de la description qui suit d'exemples de conjugués conformes à l'invention et des conditions dans lesquelles ceux-ci peuvent être utilises.

Il sera d'ailleurs fait reférence aux dessins dans lesquels:

- la fig. 1 contient des courbes représentatives des variations de l'effet cytostatique in vitro exprimées en pourcentage d'inhibition de croissance des cellules tumorales (en ordonnées), en fonction de dosages croissants, en microgrammes, de MDP-A-L seul (courbe A) ou en association avec des anticorps monoclonaux anti-MDP M-52-11 à raison de 10 microgrammes par millilitre (courbe B);
- la fig. 2 comporte des courbes représentatives de la variation de l'inhibition de la croissance des cellules tumorales (en % sur l'axe des ordonnées), en fonction de doses croissantes en microgrammes par millilitre de MDP-A-L, soit seul (courbe C), soit en présence de 10 microgrammes par millilitre de l'anticorps monoclonal M-52-11 (courbe E), soit de $F(ab)^2$ (courbe D);

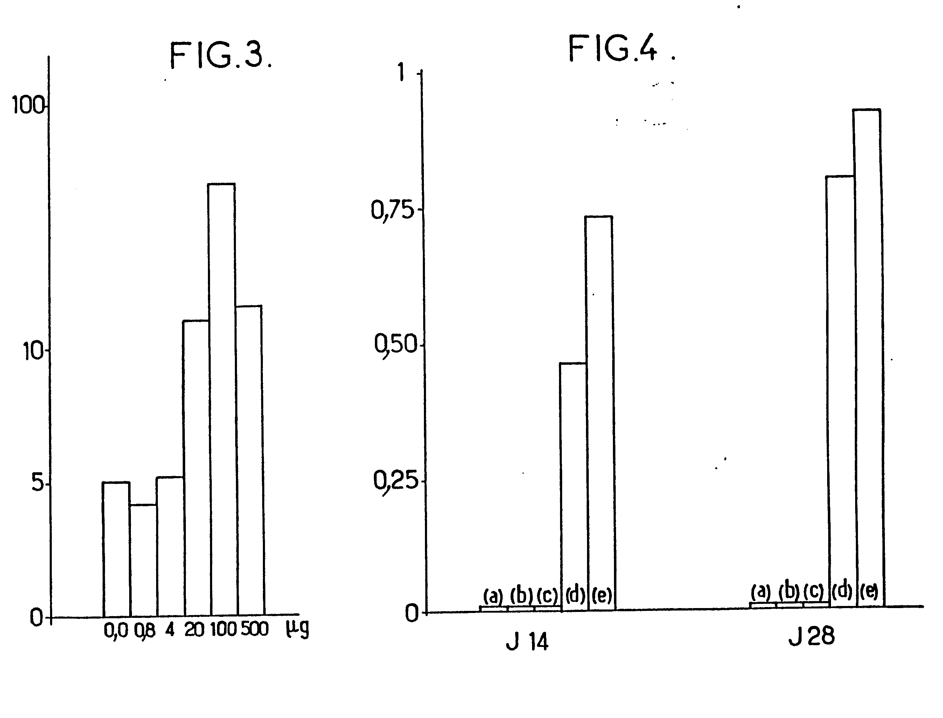

- la fig. 3 est représentative des résultats obtenus in vivo, après immunisation de l'animal d'essai avec un conjugué formé entre une albumine humaine et le MDP-lysine (50 microgrammes dans l'essai considéré) et des molécules témoins, en présence ou non d'anticorpe anti-MDP (à raison de 0 ; 0,8 ; 4 ; 20 ; 100 et 500 microgrammes respectivement), les susdits résultats étant exprimés en accroissements relatifs des taux d'anticorps mesurés contre l'albumine humaine en question;
- la fig. 4 présente des résultats d'essais analogues, dans lesquels le conjugué selon l'invention met en jeu un complexe immun MDP-A-L et des anticorps monoclonaux anti-MDP.

**EXEMPLE I:**

Les conjugués d'oligo-muramylpeptides ont été préparés à partir de multi-(poly-(N-acétyl-muramyl-L-alanyl-Disoglutaminyl)-D,L-alanyl)-poly-L-lysine, soit de façon abrégée le MDP-A-L et d'anticorps monoclonaux murins.

MDP-A-L

Le MDP-A-L a été préparé dans les conditions décrites par Chedid et Coll. 1979, Proc. Natl. Acad. Sci. U.S.A., 76, 6557. Ce produit est caractérisé par des rapports molaires MDP/Lys/Ala : 1/1/20, soit encore 0,2 mg de MDP par mg de MDP-A-L.

Anticorps monoclonaux murins

La préparation et la caractérisation d'anticorps monoclonaux anti-MDP ont été décrites dans la littérature (Bahr et Coll. 1983, Molec. Immunol.,20, 745). Dans cette étude, trois anticorps monoclonaux relatifs à des sousclasses différentes d'IgG ont été sélectionnés. Les clones retenus ont été désignés comme suit: 2-4-5 ($IgG_3$), M-52-11 ($IgG_2a$) et M-5-5 ($IgG_2b$). Ces trois anticorps monoclonaux se fixent au MDP, mais ne reconnaissent ni l'acide N-acétyl-muramique, ni le dipeptide L-alanyl-D-isoglutamine dans des essais d'inhibition de la fixation de MDP-Lys marque à la péroxydase aux anticorps anti-MDP (Bahr et Coll. 1982).

On rappellera que l'on a sélectionné ceux des clones qui ont produit des anticorps monoclonaux qui ont reconnu sélectivement le MDP-A-L, sans cependant réellement reconnaître le composé DP-A-L (c'est-à-dire le composé dans lequel le MDP est remplacé par la L-alanyl-Disoglutamine (DP)). En particulier, les dilutions limites de MDP-A-L que ces anticorps monoclonaux étaient capables de reconnaître sont plus de 100 fois plus élevées que les dilutions limites de DP-A-L et également reconnues par ces mêmes anticorps, dans les essais sur microplaques decrits dans le premier des articles sus-indiques.

Des anticorps monoclonaux de contrôle avec spécificité anti-A-L ou anti-DNP ont été préparés comme décrit dans la littérature (Eshhar et Coll., 1980 ; Behnaz-Parhami et Coll., 1983). Le précipité en sulfate d'ammonium (50 %) de fluide ascitique de chaque clone est utilisé pendant l'étude, sauf mention contraire.

Préparation de $F(ab)^2$

On fait passer les ascites contenant les immun globulines des clones M-52-11 sur une colonne du tamis moléculaire commercialisé sous la marque SEPHACRYL S-200 (PHARMACIA) et on rassemble les fractions du pic correspondant à des poids moléculaires de 160.000 daltons et capables de se fixer au MDP. On les concentre et on les dialyse à l'aide d'un tampon d'acétate de sodium 0,1 molaire, pH 4,5. On ajoute à 25 mg d'IgG dans 3 ml, 1 mg de pepsine solide (SIGMA) et on laisse incuber à 37°C pendant 18 heures. On ajuste ensuite le pH à 8,0 avec du Tris 2 M et on dialyse l'IgG digéré à l'aide de PBS à pH 7,4. On élimine le fragment Fc et l'IgG non digéré en faisant passer le préparation deux fois sur 10 ml d'une colonne de protéine A-SEPHAROSE (PHARMACIA). Le fragment F(ab)$^2$ non retenu est récupéré et contrôlé pour sa pureté selon la méthode ELISA en utilisant des antisérums de lapin spécifique de sous-classes anti-souris (Biometics). La préparation pure de F(ab)$^2$ obtenue à partir de M-52-11 a une activité identique à celle de l'IgG non digéré, vis-à-vis de la fixation au MDP-Lys-péroxydase.

Cellules

Des macrophages récoltés à partir d'exsudats péritonéaux quatre jours après une injection intrapéritonéale de 2 ml de milieu thioglycolate (INSTITUT PASTEUR, Paris) sont utilisés comme cellules effectrices. Les mastocytomes P815 utilisés comme cellules cibles sont des cellules tumorales ascitiques péritonéales provenant de souris $DBA_2$.

Préparation de monocouches de macrophages

Après récupération, on lave les macrophages et on les ensemence à ls concentration de 3,5 x 10 cellules dans 0,2 ml de milieu dans les puits de microplaque; (NUNCLON, Roskilde, Danemark). Le milieu de culture est un milieu RPMI (FLOW LABORATORIES, Rockville, Md) contenant du sérum de veau foetal inactivé à la chaleur à 10 %, de la glutamine et des antibiotiques.

On laisse incuber les cultures à 37° C, sous une atmosphère contenant du $CO_2$ 7,5 % (volume/volume). Après avoir maintenu les cellules adhérer pendant 180 mm à 37° C, donc dans des conditions leur permettant d'adhérer aux parois des puits, on enlève les cellules qui n'adhèrent pas en lavant les plaques avec du milieu 199 (INSTITUT PASTEUR, Paris).

Test d'activation des macrophages

On fait incuber des quantités variables de différentes substances avec des macrophages pendant 24 heures, ce après quoi on les enlève par lavage avant d'ajouter les cellules cibles. On ajoute ensuite 2 x $10^4$ cellules mastocytomes P815 aux monocouches de macrophages et on laisse incuber pendant 24 heures. 4 heures avant la fin de l'essai, on ajoute à chaque puits 0,1 microCl de thymidine ($^3H$). Les cellules marquées sont récupérées sur des disques en fibres de verre dans un collecteur d'échantillons multiples (SKATRON, FLOW LABORATORIES).

Estimation de la cytostase des cellules cibles

On exprime les résultats sous forme de pourcentages d'inhibition de la croissance. Ce pourcentage résulte de la formule suivante:

inhibition de croissance en % = 100 (C - A)/C.

Dans cette formule, C représente la radioactivité incorporée par les cellules de mastocytomes cultivées sur monocouches de macrophages de contrôle et A la radioactivité incorporée par les cellules mastocytomes cultivées sur monocouches de macrophages activées. On a fait trois fois l'essai pour chaque groupe d'expérimentations et les résultats obtenus sont représentatifs d'au moins deux expériences effectuées dans les mêmes conditions.

**RESULTATS**

Activation in vitro des macrophages par MDP-A-L en combinaison avec l'anti-MDP monoclonal

On fait incuber les macrophages péritonéaux pendant 24 heures avec des doses différentes de MDP-A-L seul ou en combinaison avec l'anticorps anti-MDP (clone M-52-11, 10 microgrammes/millilitre).

Comme on le voit sur la fig. 1, une concentration minimum de 1 microgramme/millilitre de MDP-A-L est nécessaire pour rendre les macrophages cytostatiques vis-à-vis ces cellules tumorales P815. Au contraire, lorsqu'on laisse incuber les macrophages avec MDP-A-L en présence de 10 microgrammes/millilitre d'anti-MDP monoclonal, une quantité aussi faible que 0,001 microgramme/millilitre de MDP-A-L est suffisante pour activer les macrophages.

Cette concentration de MDP-A-L représente

0,2 ng (4 x $10^{-10}$ mM) de MDP par millilitre. On observe des effets synergiques similaires entre MDP-A-L et anti-MDP avec 100, 10 ou 1 microgramme/millilitre d'anticorps monoclonaux (données non représentées). On note que lorsqu'il est ajouté seul l'anticorps M-52-11 n'a pas d'effet citostatique sur les macrophages.

Absence de synergie entre MDP-A-L et les anticorps F(ab)$^2$ anti-MDP

Les anticorps F(ab)$^2$, préparés comme indiqué plus haut, ont une activité identique quant à leur capacité de fixation à la MDP-Lys-péroxydase que les IgG non digérés.

On laisse incuber les macrophages pendant 24 heures avec 0,0001 à 100 microgrammes/millitre de MDP-A-L, soit seuls, soit en présence de 10 microgrammes/millilitre de M-52-11 ou de F(ab)$^2$. On peut voir d'après la fig. 2 qu'il n'y a pas de synergie entre MDP-A-L et F(ab)$^2$, contrairement à ce qui est observé avec les anticorps intacts.

Il résulte des essais qui précèdent qu'il faut au moins 1 microgramme/millilitre de MDP-A-L pour induire une activité cytostatique spécifique de macrophages péritonéaux murins.

Au contraire, en présence d'anticorps monoclonaux anti-MDP, une quantité aussi faible que 0,001 microgramme/millilitre de MDP-A-L est suffisante pour activer les macrophages. Cette concentration de MDP-A-L correspond à 0,2 ng (4 x $10^{-10}$ mM) de MDP par millilitre. Cet effet synergique avec des quantités correspondant à la limite inférieure de MDP-A-L est observé avec les anticorps monoclonaux anti-MDP de différents isotypes ($IgG_2a$, $IgG_2b$ et $IgG_3$) et également avec un anticorps monoclonal dirigé contre la chaîne

A-L. Ainsi, l'effet synergique semble être lié à la formation de complexes immuns entre MDP-A-L et les anticorps. Enfin l'activité synergique entre MDP-A-L et les anticorps spécifiques suppose que le conjugué selon l'invention comporte bien la partie Fc de l'immunoglobuline, comme le démontre l'inaptitude de $F(ab)^2$ à induire une telle activité, ce qui tend à soutenir l'hypothèse selon laquelle l'accroissement de l'activité observée implique la fixation des complexes immuns MDP sur les récepteurs Fc de la membrane des macrophages.

**EXEMPLE II:**

Des conjugués entre le MDP-lysine (N-acétyl-L-alanyl-D-isoglutaminyl-L-lysine) et une sérum-albumine humaine ont été obtenus par coupage au glutaraldéhyde. Ce couplage a été réalisé dans les conditions suivantes:

Les proportions relatives de MDP-Lys et de sérum-albumine humaine dans le produit final ont été de 16:1.

Des groupes de cinq souris ont été immunisées avec des doses égales chaque fois à 50 microgrammes du conjugué obtenu en présence de doses croissantes d'anticorps monoclonal contre le MDP.

7 jours plus tard, des échantillons de sang ont été prélevés sur les animaux des divers groupes. Leurs teneurs respectives en anticorps à l'égard de la sérum-albumine ont été testées par une méthode ELISA mettant en jeu des plaques de microtitration dont les puits avaient été revêtus de solutions contenant 10 microgrammes/ml de sérum-albumine humaine par puits. Les sérums des animaux de chaque groupe avaient été réunis et dilués au 1/100e avant d'être introduits dans les puits. Après lavage de ces puits, dans des conditions classiques, des immunoglobulines de lapin anti-souris, couplées à de la péroxydase, ont ensuite été ajoutées dans les puits. Les activités des enzymes fixées ont été mesurées par leur action sur un mélange de O-phénylène-diamine et d'eau coxygénée. Les taux d'anticorps mesurés résultent de l'examen de la fig. 3. Les barres verticales attestent de l'accroissement des taux d'anticorps mesurés (en ordonnées), en fonction des proportions en microgrammes d'anticorps anti-MDP mesurés. Comme le montre la fig. 3, l'utilisation de 20 à 500 microgrammes d'anticorps anti-MDP, au niveau de l'immunisation, conduit à une exaltation de la reponse immunitaire. En particulier, celle-ci est maximum lorsque l'immunisation a été réalisée en présence du complexe obtenu par réaction de 50µg du conjugué formé entre la sérum-albumine et le MDP-Lys et 100 microgrammes d'anticorps anti-MDP. On observe que lorsque l'immunisation a été effectuée en présence de 50 microgrammes du premier conjugué mentionné et de 500 microgrammes d'anticorps anti-MDP (soit 10 fois plus), on obtient toujours encore une reponse immunitaire plus importante que celle qui pouvait être observée après immunisation avec le conjugué albumine-MDP-Lys en l'absence d'anticorps anti-MDP ou en présence de faibles doses de ce dernier.

**EXEMPLE III:**

Des groupes de cinq souris ont été immunisées avec du MDP-A-L, en présence ou non d'anticorps anti-MDP. Ces immunisations ont été réalisées en ayant recours au protocole suivant:

a) 25 microgrammes A-L,
b) 25 microgrammes A-L + 100 microgrammes anti-MDP ascitique $IgG_2a$ (M-52-11),
c) 25 microgrammes MDP-A-L,
d) 25 microgrammes MDP-A-L + 100 microgrammes anti-MDP ascitique $IgG_3$ (2-4-5),
e) 25 microgrammes MDP-A-L + 100 microgrammes anti-MDP ascitique $IgG_2a$ (M-52-11).

Des prélèvements de sang sont effectués et les sérums des animaux de chaque groupe sont réunis aux jours et 28 respectivement après l'immunisation. Ils sont ensuite testés contre A-L par une methode ELISA sur microplaques dont les puits ont au préalable été revêtus avec une solution contenant 10 microgrammes/millilitre de A-L. Les sérums réunis, dilués au 1/400e ont été introduits dans les puits. Après incubation et lavage des puits avec des tampons appropriés, des immunoglobulines de lapin anti-souris marquées à la péroxydase diluée au 1/10.000e ont été ajoutées. L'activité enzymatique a été mesurée contre le même substrat que ci-dessus.

Les résultats sont exprimés dans la fig. 4 par des barres verticales correspondant au taux de densite optique mesuré en réponses primaire et secondaire aux jours 14 et 28, respectivement dans les cas a) à e) définis ci-dessus.

Comme dans l'exemple II, on observe une forte exaltation de l'activité stimulant du MDP-A-L, en présence d'anticorps anti-MDP.

Les compositions obtenues paraissent particulièrement bien adaptées à l'activation des macrophages vis-à-vis de preliférations tumorales. Elles doivent pouvoir être utilisées efficacement chaque fois qu'il est necessaire ou utile d'augmenter l'activité des immunomodulateurs au niveau du macrophage.

L'invention concerne également les préparations pharmaceutiques dans lesquelles les compositions à base de muramylpeptides et d'anticorps anti-muramylpeptides sont associées à un excipient pharmaceutique permettant leur administration in vivo. Ces préparations se présentent avantageusement sous forme de

solutions, suspensions ou liposomes injectables, contenant une dose efficace des éléments essentiels de la composition selon l'invention. De préférence, ces solutions, suspensions ou formes liposomes sont réalisées dans une phase aqueuse stérilisée isotonique, de préférence saline ou glucosée.

L'invention concerne plus particulièrement de telles suspensions, solutions ou formes liposomes qui sont aptes à être administrées par injections intradermiques, intramusculaires ou sous-cutanées, ou encore par scarifications.

Elle concerne également des préparations pharmaceutiques administrables par d'autres voies notamment par voie orale ou rectale, ou encore sous forme d'aérosols destinés à venir en contact avec des muqueuses, notamment les muqueuses oculaires, nasales, pulmonaires ou vaginales.

En conséquence, elle concerne des préparations pharmaceutiques dans lesquelles l'une au moins des compositions selon l'invention se trouve associée à des excipients pharmaceutiquement acceptables, solides ou liquides, adaptés à la constitution de formes orale, oculaire ou nasale ou avec des excipients adaptés à la constitution de formes d'administration rectale ou encore avec des excipients gélatineux pour l'administration vaginale. Elle concerne aussi des préparations liquides isotoniques contenant les constituants - au préalable séparés ou non - ou les immun-complexes de la composition selon l'invention, adaptées à l'administration sur les muqueuses, notamment oculaire ou nasale. Elle concerne enfin des compositions formées de gaz liquéfiés pharmaceutiquement acceptables, dans lesquels les constituants de la composition selon l'invention ou les immun-complexes formés entre ces constituants sont dissous ou maintenus en suspension, et dont la détente provoque la dispersion en un aérosol.

Lorsque les compositions selon l'invention sont utilisées en association avec un antigène vaccinant elles contiennent avantageusement en outre un véhicule, tel que la polyvinyl-pyrrolidone, facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthyl-cellulose, les hydroxydes et phosphates d'aluminium ou tous autres adjuvants de ce type, bien connus de l'homme de l'art.

Les préparations pharmaceutiques selon l'invention sont essentiellement destinées à moduler l'activité des macrophages. Elles sont utiles chaque fois que l'on cherche à provoquer une immunité à médiation cellulaire à l'égard d'antigènes pathogènes (par exemple de nature bactérienne, virale ou parasitaire) ou contre des proliférations des cellules tumorales. Sans que les indications qui suivent puissent avoir un caractere limitatif, on mentionnera que les doses unitaires actives du médicament, rapportées aux muramylpeptides peuvent être de l'ordre de 1 à 100 microgrammes par kg. De préférence, les proportions relatives de muramylpeptide et d'anticorps peuvent être de l'ordre de 1 molécule de muramylpeptide (ou motif muramylpeptide) pour 10 à 100 molécules d'anticorps.

L'invention concerne encore plus particulèrement des préparations pharmaceutiques dans lesquelles le muramylpeptide ou l'oligo-muramylpeptide, d'une part, et la molécule-vecteur, d'autre part, sont en fait présentées dans des conditionnements séparés: elles comportent par exemple un flacon contenant l'oligo-muramylpeptide et un flacon combinant la molécule-vecteur. Cette forme de présentation est particulièrement avantageuse puisque la réaction entre les deux constituants est en général spontanée à l'occasion de leur mise en contact, de sorte que l'immun-complexe selon l'invention peut alors être formé extemporanément au moment de l'usage. Les deux constituants sont mélangés et dissous (ou vice versa) dans une solution injectable. Il est préférable de laisser reposer la solution formée pendant quelque temps, par exemple 10 minutes à la température ambiante, avant l'administration, par exemple par injection. Les proportions relatives des deux constituants sont dans un tel cas avantageusement conformes à celles qui permettent l'obtention d'une composition finale comportant les proportions relatives en muramylpeptides et en anticorps contenus dans les intervalles qui ont été indiqués ci-dessus à titre d'exemple.

L'invention concerne de même encore les compositions du type sus-indiqué, présentées en conditionnements séparés, les constituants qu'ils contiennent étant le cas échéant destinés à être administrés ensemble ou séparément dans le temps, en conformité avec un protocole d'administration pré-établi en vue d'obtenir la manifestation, avec le maximum d'efficacité de leurs effets thérapeutiques du type indiqué plus haut. Par exemple, les anticorps anti-muramylpeptide sont 5 destinés à être administrés avant le muramylpeptide lui-même ou vice versa. L'invention concerne alors plus particulièrement les présentations de ces conditionnements sous des emballages appropriés et, le cas échéant, avec notices générales d'utilisation, en ce qui concerne les intervalles de temps devant s'écouler entre les administrations décalées des constituants séparés. Ces conditionnements peuvent contenir des doses unitaires efficaces des constituants des préparations pharmaceutiques selon l'invention.

Les compositions selon l'invention, notamment les immuns-complexes, peuvent également être pré-conditionnées à l'état lyophilisé. Il peut là encore s'agir de doses unitaires pré-établies, en vue de la préparation de solutions extemporanées. Il est remarqué que les doses administrables sont à ce point faiblesque, notamment dans le cas de compositions administrables par injection, leur mise en solution dans les volumes de liquide requis pour l'injection ne pose en général aucun problème.

Il va sans dire que les pré-conditionnements pharmaceutiques ainsi obtenus entrent dans le cadre des revendications qui suivent et relatives à des compositions pour la stimulation des macrophages in vivo.

L'invention concerne encore les réactifs biologiques que constituent les compositions à base de muramylpeptides et d'anticorps selon l'invention. Les compositions selon l'invention peuvent également être

utiles pour la fabrication d'anticorps efficaces et en quantités importantes contre un antigène déterminé, par immunisation d'un animal avec un conjugué préalablement formé, conformément aux enseignements de cette invention. L'antigène est alors utilisée comme "molécule-porteuse", celle-ci étant associée avec à la fois un muramylpeptide et un anticorps répondant aux définitions qui ont été données plus haut.

L'invention concerne enfin un procédé d'activation in vivo des macrophages, ce procédé étant caractérisé par l'administration à un hôte de doses efficaces, soit simultanément soit à des instants distincts d'un muramylpeptide et d'anticorps dirigés contre ce muramylpeptide, soit encore d'une dose efficace d'une composition contenant l'un et l'autre et, dans laquelle, le cas échéant, l'immun-complexe entre le muramylpeptide et l'anticorps aura été préformé.

Il va sans dire que, dans le cas où les administrations du muramylpeptide et des anticorps sont décalées, ce décalage ne doit pas excéder celui gui aurait pour effet de réduire l'effet d'exaltation recherché de l'activation des macrophages par le muramylpeptide.

L'invention concerne enfin un procédé d'utilisation de la composition selon l'invention ou de ses constituants essentiels (muramylpeptide et anticorps) pour la préparation d'une composition pharmaceutique utilisable pour le traitement de maladies sensibles à l'action de macrophages activés et par conséquent susceptibles d'être combattues par des compositions ayant la capacité d'activer des macrophages ou encore pour la préparation de compositions destinées à la vaccination contre un agent pathogène déterminé ou à l'utilisation conjointe avec un vaccin contre ledit agent pathogène.

**Revendications**

pour les états contractants BE, CH, DE, GB, IT, LI, LU, NL, SE.

1. Composition associant un muramylpeptide ou oligo-muramylpeptide, combine ou non avec un antigène ou un haptène porteur d'un site antigénique, caractéristique de cet haptène, et un anticorps dirigé contre ce muramylpeptide et apte à former avec lui un immun-complexe pour l'utilisation en vue de l'activation in vivo des macrophages.

2. Composition selon la revendication 1, caractérisée en ce que l'anticorps est monoclonal et dirigé préférentiellement contre l'ensemble de la structure muramylpeptide.

3. Composition selon la revendication 2, caractérisée en ce que l'anticorps monoclonal n'est que peu actif ou même inactif à l'égard de la structure de l'acide N-acétyl muramique ou de celle du peptide entrant dans la constitution dudit muramylpeptide.

4. Conjugué selon l'une quelconque des revendications 1 à 3, caractérisé en ce que l'oligo-muramylpeptide est un oligomère du muramylpeptide correspondant.

5. Conjugué selon l'une quelconque des revendications 1 à 3, caractérisé en ce que l'oligo-muramylpeptide comporte une molécule porteuse sur laquelle sont fixées une ou plusieurs molécules de muramylpeptide.

6. Composition selon l'une quelconque des revendications 1 à 5, caractérisée en ce que le muramylpeptide ou le muramylpeptide monomère dont est dérivé le susdit oligo-muramylpeptide est caractérisé par la formule suivante:

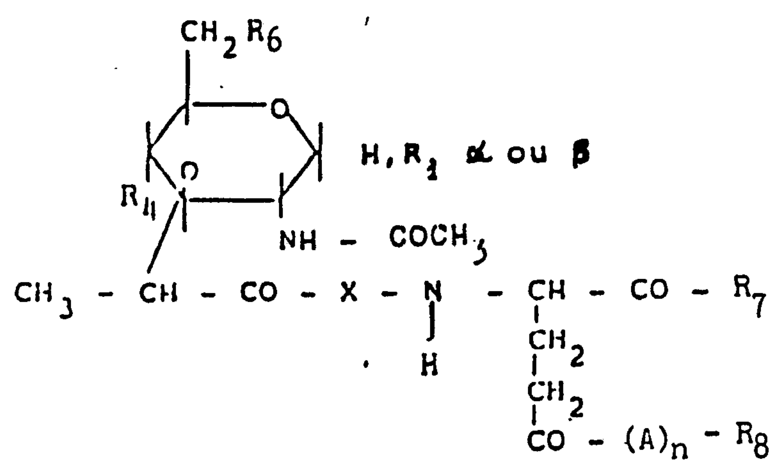

dans laquelle les substituants $R_1$, $R_4$, $R_6$, X, $R_7$, $R_8$, A et n ont respectivement les significations suivantes:

- $R_1$ est $-NH_2$, -OH, -NY ou -OY, Y représentant un groupe substitué ou non par un groupe amino, ce groupe étant choisi parmi alcoyle, aryle ou alcoyl-aryle portant un maximum de 10 atomes de carbone;
- $R_4$ est hydroxyle, acyloxy ou alcoxy ayant au plus 4 atomes de carbone, ou monosuccinyle;

- $R_6$ est $-NH_2$, -OH,-NHZ ou -OZ, Z étant un radical linéaire ou ramifié formé par acyle ou alkyle contenant de 1 à 90 atomes de carbone et portant, le cas échéant, des substituants hydroxyle, carboxyle, carbonyle, amino, cyclopropyle ou méthoxyle;
- X est un résidu aminoacyle du groupe comprenant: alanyle, arginyle, asparagyle, aspartyle, cystéinyle, glutaminyle, glutamyle, glycyle, histidyle, hydroxypropyle, isoleucyle, leucyle, lysyle, méthionyle, phénylalanyle, propyle, séryle, thréonyle, tryptophanyle, tyrosyle et valyle, N-méthyl-alanyle;
- $R_7$ est un groupe -OH $-NH_2$, alcoylamide, alcoxy linéaire ou ramifié, aryle ou alcoxy-aryle comprenant au plus 10 atomes de carbone,
- $R_8$ est un groupe -OH, $-NH_2$ ou un groupe alcoxy comprenant de 1 à 10 atomes de carbone,
- A représente un acide aminé tel qu'un résidu alanyle, lysine glutamine et
- n est égal à 0,1 ou 2.

7. Composition selon la revendication 6, caractérisée en ce que les acides aminés intervenant dans la constitution de la chaîne peptidique sont (à l'exception de la glycine) lévogyres.

8. Composition selon la revendication 6 ou 7, caractérisée en ce que la molécule porteuse est constituée par un antigène, une molécule ayant des propriétés immunogènes ou un haptène portant un déterminant antigénique caracteristique de cet antigène ou de cette molécule immunogène.

9. Préparation pour la stimulation des macrophages in vivo, caractérisée en ce qu'elle contient, en association avec un véhicule biologique permettant l'administration de ladite composition in vivo, une composition selon l'une quelconque des revendications 1 à 8.

10. Préparation pour la stimulation de la reponse immunitaire in vivo contre un antigène déterminé, caractérisée en ce qu'elle contient en association avec un véhicule biologique permettant son administration in vivo, une composition selon l'une quelconque des revendications 1 à 8 et un agent immunogène apte à induire in vivo la production d'anticorps actifs contre le susdit antigène.

11. Composition ou préparation selon l'une quelconque des revendications 1 à 10, caractérisée en ce que les proportions relatives de muramylpeptides et d'anticorps sont de l'ordre de 1 molécule de muramylpeptide pour 10 à 100 molécules d'anticorps.

12. Composition ou préparation selon l'une quelconque des revendications 1 à 11, caractérisée par le fait qu'elle se presente sous forme de doses unitaires en équivalent muramylpeptide de 1 à 100 microgramme/kg.

**Revendications**

pour l'état contractant AT.

1. Procédé de préparation d'une composition pour l'utilisation en vue de l'activation in vivo des macrophages, caractérisé en ce que l'on associe avec un excipient pharmaceutique un immuno-complexe préalablement formé entre un muramyl-peptide ou oligo-muramylpeptide, combiné ou non avec un antigène ou un haptène porteur d'un site antigénique caractéristique de cet haptène, d'une part, et un anticorps dirigé contre ce muramyl-peptide et apte à former avec lui cet immuno-complexe.

2. Procédé selon la revendication 1, caractérisé en ce que l'anticorps est monoclonal et dirigé préférentiellement contre l'ensemble de la structure muramylpeptide.

3. Procédé selon la revendication 2, caractérisé en ce que l'anticorps monoclonal entrant dans l'immunocomplexe n'est que peu actif ou même inactif à l'égard de la structure de l'acide N-acétyl muramique ou de celle du peptide entrant dans la constitution dudit muramylpeptide.

4. Procédé selon l'une quelconque des revendications 1 à 3 caractérisé en ce que l'oligo-muramylpeptide entrant dans l'immuno-complexe est un oligomère du muramylpeptide correspondant.

5. Procédé selon l'une quelconque des revendications 1 à 3, caractérisé en ce que l'oligo-muramylpeptide entrant dans l'immuno-complexe comporte une molécule porteuse sur laquelle sont fixées une ou plusieurs molécules de muramylpeptides.

6. Procédé selon l'une quelconque des revendications 1 à 5, caractérisé en ce que le muramylpeptide ou le muramylpeptide monomère dont est dérivé le susdit oligo-muramylpeptide entrant dans l'immuno-complexe est caractérisé par la formule suivante:

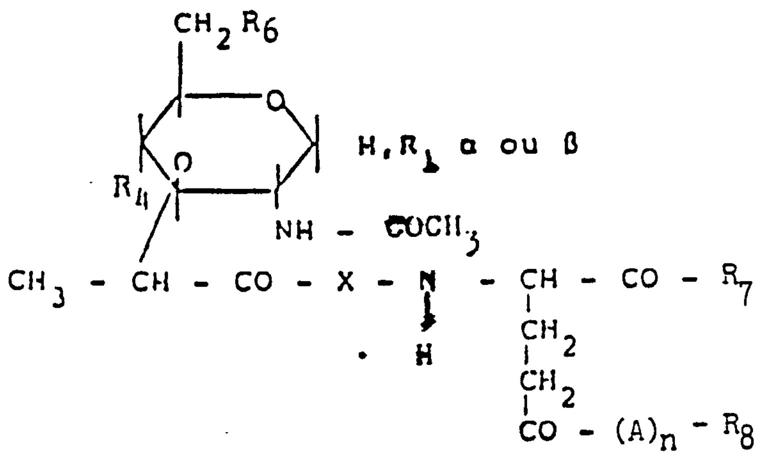

dans laquelle les substituants $R_1$, $R_4$, $R_6$, X, $R_7$, $R_8$, A et n ont respectivement lea significations suivantes:

- $R_1$ est $-NH_2$; -OH, -NY ou -OY, Y représentant un groupe substitué ou non par un groupe amino, ce groupe étant choisi parmi alcoyle, aryle ou alcoyl-aryle portant un maximum de 10 atomes de carbone;
- $R_4$ est hydroxyle, acyloxy ou alcoxy ayant au plus 4 atomes de carbone, ou monosuccinyle;
- $R_6$ est $-NH_2$, -OH,-NHZ ou -OZ, Z étant un radical linéaire ou ramifié formé par acyle ou alkyle contenant de 1 à 90 atomes de carbone et portant, le cas échéant, des substituants hydroxyle, carboxyle, carbonyle, amino, cyclopropyle ou méthoxyle;
- X est un résidu aminoacyle du groupe comprenant: alanyle, arginyle, asparagyle, aspartyle, cystéinyle, glutaminyle, glutamyle, glycyle, histidyle, hydroxypropyle, isoleucyle, leucyle, lysyle, méthionyle, phényl-alanyle, propyle, séryle, thréonyle, tryptophanyle, tyrosyle et valyle, N-méthyl-alanyle;
- $R_7$ est un groupe -OH, $-NH_2$, alcoylamide, alcoxy linéaire ou ramifié, aryle ou alcoxy-aryle comprenant au plus 10 atomes de carbone,
- $R_8$ est un groupe -OH, $-NH_2$ ou un groupe alcoxy comprenant de 1 à 10 atomes de carbone,
- A représente un acide aminé tel qu'un résidu alanyle, lysine, glutamine et
- n est égal à 0,1 ou 2.

7. Procédé selon la revendication 6, caractérisé en ce que les acides aminés intervenant dans la constitution de la chaîne peptidique du muramylpeptide entrant dans l'immuno-complexe sont (à l'exception de la glycine) lévogyres.

8. Procédé selon la revendication 6 ou 7, caractérisé en ce que la molécule porteuse associée avec le muramylpeptide entrant dans l'immuno-complexe est constituée par un antigène, une molécule ayant des propriétés immunogènes ou un haptène portant un déterminant antigénique caractéristique de cet antigène ou de cette molécule immunogène.

9. Procédé de préparation d'une composition selon l'une quelconque des revendications 1 à 8 capable d'exercer en outre une stimulation de la réponse immunitaire in vivo contre un antigène déterminé, caractérisé en ce que l'on associe également à cette composition un agent immunogène apte à induire in vivo la production d'anticorps actifs contre le susdit antigène.

10. Procédé selon l'une quelconque des revendications 1 à 9, caractérisé en ce que l'on associe ledit muramylpeptide ou oligomuramylpeptide avec l'anticorps dans des proportions relatives de muramylpeptides et d'anticorps, de l'ordre de 1 molécule de muramylpeptide pour 10 à 100 molécules d'anticorps.

11. Procédé selon l'une quelconque des revendications 1 à 10, caractérisé par le fait que l'on dose la composition sous forme de doses unitaires en équivalent muramyl-peptide de 1 à 100 microgramme/kg.

**Patentansprüche**

für die Vertragsstaaten BE, CH, DE, GB, IT, LI, LU, NL, SE.

1. Zusammensetzung zur Verbindung eines Muramylpeptids oder Oligo-Muramylpeptids, das gegebenenfalls mit einem Antigen oder einem Trägerhapten eines für dieses Hapten charakteristischen Antigen-Sites und einem gegen das Muramylpeptid gerichteten Antikörper verbunden ist, der fähig ist, mit diesem einen Immunkomplex zur Verwendung bei der Aktivierung in vivo von Makrophagen zu bilden.

2. Zusammensetzung nach Anspruch 1, dadurch gekennzeichnet, daß der Antikörper monoklonal ist und vorzugsweise gegen die gesamte Muramylpeptid-Struktur gerichtet ist.

3. Zusammensetzung nach Anspruch 2, dadurch gekennzeichnet, daß der monoklonale Antikörper gegenüber der Struktur der N-Muramacetylsäure oder dem des in die Struktur des Muramylpeptids eintretenden Peptid nur wenig aktiv oder sogar inaktiv ist.

4. Verbindung nach einem der Ansprüche 1 bis 3, dadurch gekennzeichnet, daß das Oligo-Muramylpeptid ein Oligomer des entsprechenden Muramylpeptids ist.

5. Verbindung nach einem der Ansprüche 1 bis 3, dadurch gekennzeichnet, daß das Oligo-Muramylpeptid ein Trägermolekül aufweist, an das ein oder mehrere Muramylpeptid-Moleküle gebunden sind.

6. Zusammensetzung nach einem der Ansprüche 1 bis 5, dadurch gekennzeichnet, daß das Muramylpeptid oder das Muramylpeptid-Monomer, von dem das Oligo-Muramylpeptid abgeleitet ist, durch die folgende Formel gekennzeichnet ist:

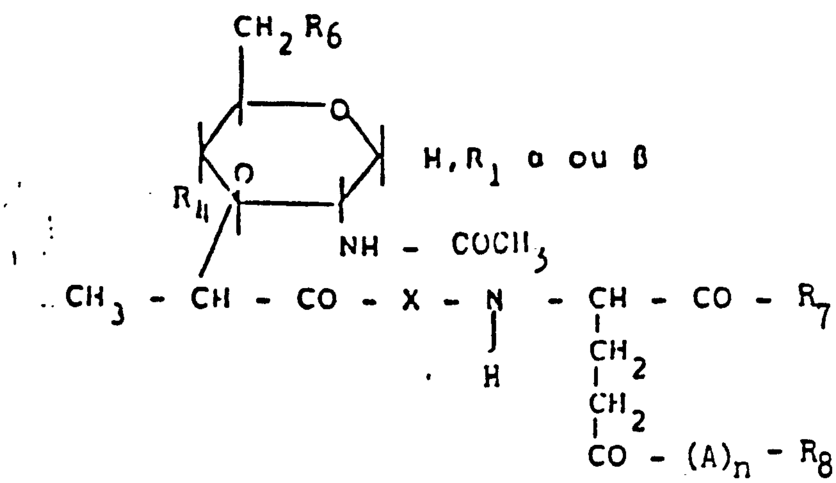

wobei die Substituenten $R_1$, $R_4$, $R_6$, X, $R_7$, $R_8$, A und n jeweils folgende Bedeutung haben:

- $R_1$ bedeutet $-NH_2$, -OH, -NY oder -OY, wobei Y eine Gruppe darstellt, die gegebenenfalls durch eine Aminogruppe, ausgewählt aus einer Gruppe bestehend aus Alcoyl, Aryl oder Alcoyl-Aryl mit maximal 10 Kohlenstoffatomen substituiert ist;
- $R_4$ bedeutet Hydroxyl, Acyloxy oder Alcoxy mit maximal 4 Kohlenstoffatomen, oder Monosuccinyl;
- $R_6$ bedeutet $-NH_2$, -OH, -NHZ oder -OZ, wobei Z ein linearer oder verzweigter Rest ist, oder Acyl oder Alkyl mit 1 bis 90 Kohlenstoffatomen und gegebenenfalls durch Hydroxyl, Carboxyl, Carbonyl, Amino, Cyclopropyl oder Methoxyl substituiert ist;
- X ist ein Aminoacylrest, ausgewählt aus der Gruppe bestehend aus Alanyl, Arginyl, Asparaginyl, Aspartyl, Cysteinyl, Glutaminyl, Glutamyl, Glycyl, Histidyl, Hydroxypronyl, Isoleucyl, Leucyl, Lysyl, Methionyl, Phenylalanyl, Propyl, Seryl, Threonyl, Tryptophanyl, Tyrosyl und Valyl, N-Methyl-Alanyl;
- $R_7$ bedeutet eine Gruppe -OH, $-NH_2$, Alkylamid, lineares oder verzweigtes Alcoxy, Aryl oder Alcoxy-Aryl mit höchstens 10 Kohlenstoffatomen,
- $R_8$ bedeutet eine Gruppe -OH, $-NH_2$ oder eine Alcoxygruppe mit 1 bis 10 Kohlenstoffatomen,
- A bedeutet eine Aminosäure, wie zum Beispiel einen Alanyl-, Lysin-, oder Glutaminrest und
- n ist gleich 0,1 oder 2.

7. Zusammensetzung nach Anspruch 6, dadurch gekennzeichnet, daß die in die Struktur der Peptidkette eintretenden Aminosäuren (mit Ausnahme des Glycins) linksdrehend sind.

8. Zusammensetzung nach Anspruch 6 oder 7, dadurch gekennzeichnet, daß das Trägermolekül aus einem Antigen, einem immunogene Eigenschaften aufweisenden Molekül oder einem Hapten besteht, das einen für dieses Antigen oder dieses immunogene Molekül charakteristischen Antigen-Determinanten trägt.

9. Präparat zur Stimulierung in vivo von Makrophagen, dadurch gekennzeichnet, daß es, in Verbindung mit einer die Verabreichung in vivo der Zusammensetzung ermöglichenden biologischen Trägersubstanz, eine Zusammensetzung nach einem der Ansprüche 1 bis 8 enthält.

10. Präparat zur Stimulierung in vivo der Immunreaktion gegen ein bestimmtes Antigen, dadurch gekennzeichnet, daß es, in Verbindung mit einer seine Verabreichung in vivo ermöglichenden biologischen Trägersubstanz, eine Zusammensetzung nach einem der Ansprüche 1 bis 8 und einen immunogenen Wirkstoff enthält, der fähig ist, die Produktion von aktiven Antikörpern gegen das Antigen in vivo zu induzieren.

11. Zusammensetzung oder Präparat nach einem der Ansprüche 1 bis 10, dadurch gekennzeichnet, daß das Verhältnis der Mengenanteile von Muramylpeptiden und Antikörpern in einer Größenordnung von 1 Muramylpeptid-Molekül pro 10 bis 100 Antikörper-Moleküle liegt.

12. Zusammensetzung oder Präparat nach einem der Ansprüche 1 bis 11, dadurch gekennzeichnet, daß sie oder es in einer Einheitsdosis entsprechend einem Muramylpeptid-Äquivalent von 1 bis 100 Mikrogramm/kg verabreicht wird.

**Patentansprüche**

für den Vertragsstaat AT.

1. Verfahren zur Herstellung einer Zusammensetzung zur Verwendung bei der Aktivierung in vivo von Makrophagen, dadurch gekennzeichnet, daß man mit einer pharmazeutischen Trägersubstanz einen Immunkomplex, der vorher zwischen einem Muramylpeptid oder Oligo-Muramylpeptid gebildet ist, das gegebenenfalls mit einem Antigen oder einem Trägerhapten eines für dieses Hapten charakteristischen Antigen-Sites verbunden ist und einen gegen das Muramylpeptid gerichteten Antikörper verbindet, der fähig ist, mit diesem einen Immunkomplex zu bilden.

2. Verfahren nach Anspruch 1, dadurch gekennzeichnet, daß der Antikörper monoklonal ist und vorzugsweise gegen die gesamte Muramylpeptid-Struktur gerichtet ist.

3. Verfahren nach Anspruch 2, dadurch gekennzeichnet, daß der in den Immunkomplex eintretende monoklonale Antikörper gegenüber der Struktur der N-Muramacetylsäure oder dem des in die Struktur des Muramylpeptids eintretenden Peptid nur wenig aktiv oder sogar inaktiv ist.

4. Verfahren nach einem der Ansprüche 1 bis 3, dadurch gekennzeichnet, daß das in den Immunkomplex eintretende Oligo-Muramylpeptid ein Oligomer des entsprechenden Muramylpeptids ist.

5. Verfahren nach einem der Ansprüche 1 bis 3, dadurch gekennzeichnet, daß das in den Immunkomplex eintretende Oligo-Muramylpeptid ein Trägermolekül aufweist, an das ein oder mehrere Muramylpeptid-Moleküle gebunden sind.

6. Verfahren nach einem der Ansprüche 1 bis 5, dadurch gekennzeichnet, daß das Muramylpeptid oder das Muramylpeptid-Monomer, von dem das in den Immunkomplex eintretende Oligo-Muramylpeptid abgeleitet ist, durch die folgende Formel gekennzeichnet ist:

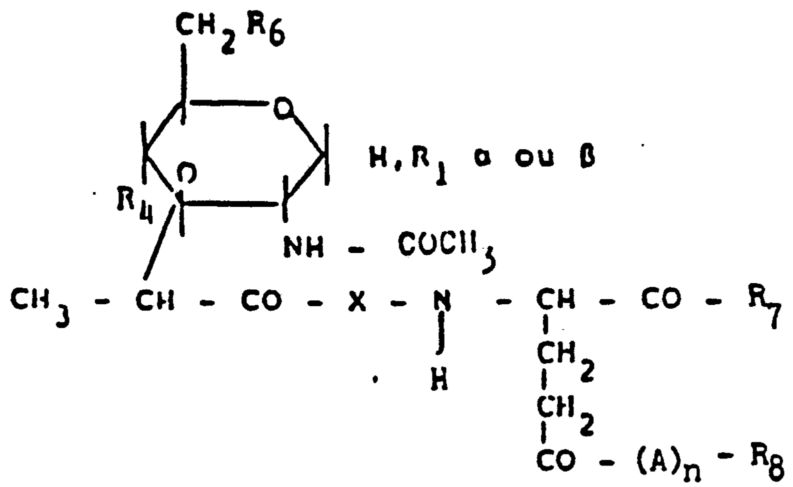

wobei die Substituenten $R_1$, $R_4$, $R_6$, X, $R_7$, $R_8$, A und n jeweils folgende Bedeutung haben:

- $R_1$ bedeutet $-NH_2$, -OH, -NY oder -OY, wobei Y eine Gruppe darstellt, die gegebenenfalls durch eine Aminogruppe, ausgewählt aus einer Gruppe bestehend aus Alcoyl, Aryl oder Alcoyl-Aryl mit maximal 10 Kohlenstoffatomen substituiert ist;
- $R_4$ bedeutet Hydroxyl, Acyloxy oder Alcoxy mit maximal 4 Kohlenstoffatomen, oder Monosuccinyl;
- $R_6$ bedeutet $-NH_2$, -OH, -NHZ oder -OZ, wobei Z ein linearer oder verzweigter Rest ist, oder Acyl oder Alkyl mit 1 bis 90 Kohlenstoffatomen und gegebenenfalls durch Hydroxyl, Carboxyl, Carbonyl, Amino, Cyclopropyl oder Methoxyl substituiert ist;
- X ist ein Aminoacylrest, ausgewählt aus der Gruppe bestehend aus Alanyl, Arginyl, Asparaginyl, Aspartyl, Cysteinyl, Glutaminyl, Glutamyl, Glycyl, Histidyl, Hydroxypronyl, Isoleucyl, Leucyl, Lysyl, Methionyl, Phenylalanyl, Propyl, Seryl, Threonyl, Tryptophanyl, Tyrosyl und Valyl, N-Methyl-Alanyl;
- $R_7$ bedeutet eine Gruppe -OH, $-NH_2$, Alkylamid, lineares oder verzweigtes Alcoxy, Aryl oder Alcoxy-Aryl mit höchstens 10 Kohlenstoffatomen,
- $R_8$ bedeutet eine Gruppe -OH, $-NH_2$ oder eine Alcoxygruppe mit 1 bis 10 Kohlenstoffatomen,
- A bildet eine Aminosäure, wie zum Beispiel einen Alanyl-, Lysin- oder Glutaminrest und
- n ist gleich 0,1 oder 2.

7. Verfahren nach Anspruch 6, dadurch gekennzeichnet, daß die in die Struktur der Peptidkette des in den Immunkomplex eintretenden Muramylpeptids eintretenden Aminosäuren (mit Ausnahme des Glycins) linksdrehend sind.

8. Verfahren nach Anspruch 6 oder 7, dadurch gekennzeichnet, daß das mit dem in den Immunkomplex eintretenden Muramylpeptid verbundene Trägermolekül aus einem Antigen, einem immunogene Eigenschaften aufweisenden Molekül oder einem Hapten besteht, das einen für dieses Antigen oder dieses immunogene

Molekül charakteristischen Antigen-Determinanten trägt.

9. Verfahren zur Herstellung einer Zusammensetzung nach einem der Ansprüche 1 bis 8, die unter anderem fähig ist, die Stimulierung in vivo der Immunreaktion gegen ein bestimmtes Antigen auszuführen, dadurch gekennzeichnet, daß man diese Zusammensetzung in gleicher Weise mit einem immunogenen Wirkstoff verbindet, der fähig ist, die Produktion von aktiven Antikörpern gegen das Antigen in vivo einzuleiten.

10. Verfahren nach einem der Ansprüche 1 bis 9, dadurch gekennzeichnet, daß man das Muramylpeptid oder Oligo-Muramylpeptid mit dem Antikörper verbindet, wobei das Verhältnis der Mengenanteile von Muramylpeptiden und Antikörpern in einer Größenordnung von 1 Muramylpeptid-Molekül pro 10 bis 100 Antikörper-Moleküle liegt.

11. Verfahren nach einem der Ansprüche 1 bis 10, dadurch gekennzeichnet, daß die Zusammensetzung in einer Einheitsdosis entsprechend einem Muramylpeptid-Äquivalent von 1 bis 100 Mikrogramm/kg verabreicht wird.

## Claims

for the contracting states BE, CH, DE, GB, IT, LI, LU, NL, SE.

1. A composition comprising a muramylpeptide or oligo-muramylpeptide, combined or not combined with an antigen or with a hapten carrier of an antigenic site, characteristic of this hapten, and an antibody specific against this muramylpeptide and capable of forming therewith an immuno-complex for use in the in vivo activation of macrophages.

2. A composition according to claim 1, characterized in that the anitbody is monoclonal and preferentially specific against the whole muramylpeptide structure.

3. A composition according to claim 2, characterized in that the monoclonal antibody is only slightly active or even inactive with respect to the structure of Nacetyl muramic acid or that of the peptide forming part of the constitution of the muramylpeptide.

4. A conjugate according to any of claims 1 to 3, characterized in that the oligo-muramylpeptide is an oligomer of the corresponding muramylpeptide.

5. A conjugate according to any of claims 1 to 3, characterized in that the oligo-muramylpeptide includes a carrier molecule on which one or more muramylpeptide molecules are attached.

6. A composition according to any of claims 1 to 5, characterized in that the muramylpeptide or the muramylpeptide monomer from which the said oligomuramylpeptide is derived is characterized by the following formula:

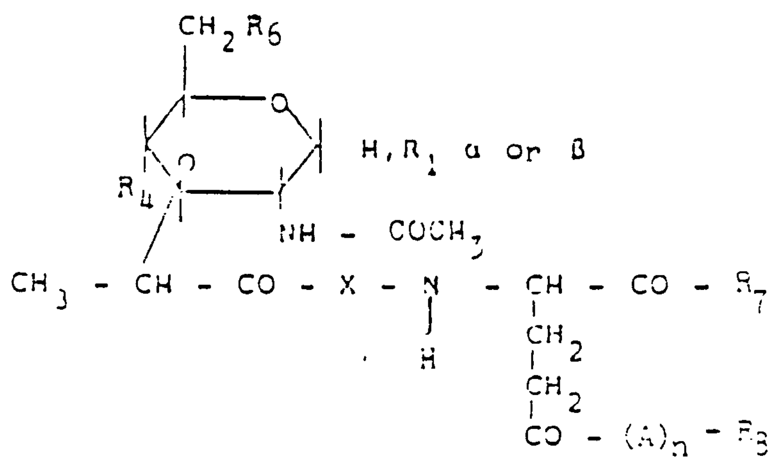

in which the substituents $R_1$, $R_4$, $R_6$, X, $R_7$, $R_8$, A and n respectively have the following meanings:

- $R_1$ is $-NH_2$, -OH, -NY or -OY, Y representing an amino-substituted or -unsubstituted alkyl, aryl or alkaryl group containing a maximum of 10 carbon atoms;
- $R_4$ is hydroxy, acyloxy or alkoxy having at most 4 carbon atoms, or monosuccinyl;
- $R_6$ is $-NH_2$, -OH, -NHZ or -OZ, Z being a straight- or branched-chain radical constituted by acyl or alkyl containing from 1 to 90 carbon atoms and bearing, if desired, hydroxyl, carboxyl, carbonyl, amino, cyclopropyl or methoxy substituents;
- X is an aminoacyl residue selected from the group comprising:

alanyl, arginyl, asparagyl, aspartyl, cysteinyl, glutaminyl, glutamyl, glycyl, histidyl, hydroxy-propyl, isoleucyl, leucyl, lysyl, methionyl, phenyl-alanyl, propyl, seryl, threonyl, tryptophenyl, tyrosyl and valyl, N-methyl-alanyl;

- $R_7$ is an -OH, -$NH_2$, alkylamide, straight- or branched-chain alkoxy, aryl or alkoxy-aryl group containing at most 10 carbon atoms;
- $R_8$ is an -OH, -$NH_2$ or alkoxy group containing from 1 to 10 carbon atoms;
- A represents an amino acid such as an alanyl, lysine [or] glutamine residue; and
- n is equal to 0,1 or 2.

7. Composition according to claim 6, characterized in that the amino acids incorporated in the constitution of the peptide chain are (with the exception of glycine) laevorotatory.

8. Composition according to claim 6 or 7, characterized in that the carrier molecule is constituted by an antigen, by a molecule having immunogenic properties or by a hapten bearing an antigenic determinant characteristic of this antigen or of this immunogenic molecule.

9. Preparation for the stimulation of macrophages in vivo, characterized in that it contains a composition according to any of claims 1 to 8 in association with a biological vehicle enabling the administration of the said composition in vivo.

10. Preparation for the stimulation of the immune response in vivo against a specific antigen, characterized in that it contains a composition according to any of claims 1 to 8 and an immunogenic agent capable of inducing in vivo the production of antibodies active against the said antigen, in association with a biological vehicle enabling its administration in vivo.

11. Composition or preparation according to any of claims 1 to 10, characterized in that the relative proportions of the muramylpeptide and of the antibody are of the order of 1 molecule of muramylpeptide per 10 to 100 molecules of antibody.

12. Composition or preparation according to any of claims 1 to 11, characterized by the fact that it is presented in the form of unit doses of 1 to 100 micrograms/kg of muramylpeptide equivalent.

**Claims**

for the contracting state AT.

1. Process for preparation of a composition for employment in the activation of macrophages in vivo, characterized in that with a pharmaceutical excipient one associates an immuno-complex previously-formed between on the one hand a muramylpeptide or oligomuramylpeptide, combined or not combined with an antigen or with a hapten bearing an antigen site characteristic of this hapten, and an antibody specific against this muramylpeptide and capable of forming this immunocomplex with it.

2. Process according to claim 1, characterized in that the antibody is monoclonal and preferentially specific against the whole of the muramylpeptide structure.

3. Process according to claim 2, characterized in that the monoclonal antibody forming part of the immuno-complex is only slightly active or even inactive with respect to the structure of the N-acetyl muramic acid or of that of the peptide forming part of the constitution of the said muramylpeptide.

4. Process according to any of claims 1 to 3, characterized in that the oligo-muramylpeptide forming part of the immuno-complex is an oligomer of the corresponding muramylpeptide.

5. Process according to any of claims 1 to 3, characterized in that the oligo-muramylpeptide forming part of the immuno-complex includes a carrier molecule on which one or more muramylpeptide molecules are attached.

6. Process according to any of claims 1 to 5, characterized in that the muramylpeptide or the monomeric muramylpeptide from which the said oligo-muramylpeptide is derived that forms part of the immuno-complex is characterized by the following formula:

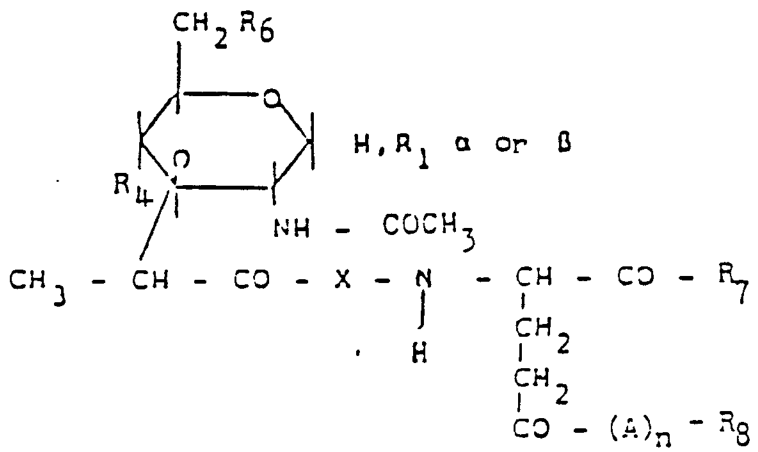

in which the substituents $R_1$, $R_4$, $R_6$, X, $R_7$, $R_8$, A and n respectively have the following meanings:

- $R_1$ is $-NH_2$, -OH, -NY or -OY, Y representing an amino-substituted or -unsubstituted group selected from alkyl, aryl or alkaryl having a maximum of 10 carbon atoms;
- $R_4$ is hydroxy, acyloxy or alkoxy having at most 4 carbon atoms, or monosuccinyl;
- $R_6$ is $-NH_2$, -OH, -NHZ or -OZ, Z being a straight- or branched-chain radical constituted by acyl or alkyl containing from 1 to 90 carbon atoms and bearing, if desired, hydroxy, carboxyl, carbonyl, amino, cyclopropyl or methoxy substituents;
- X is an aminoacyl residue selected from the group comprising:
alanyl, arginyl, asparagyl, aspartyl, cysteinyl, glutaminyl, glutamyl, glycyl, histidyl, hydroxy-propyl, isoleucyl, leucyl, lysyl, methionyl, phenyl-alanyl, propyl, seryl, threonyl, tryptophanyl, tyrosyl and valyl, N-methyl-alanyl;
- $R_7$ is an -OH, $-NH_2$, alkylamide, straight- or branched-chain alkoxy, aryl or alkoxy-aryl group comprising at most 10 carbon atoms;
- $R_8$ is an -OH, $-NH_2$ or alkoxy group comprising from 1 to 10 carbon atoms;
- A represents an amino acid such as an alanyl, lysine (or) glutamine residue; and
- n is equal to 0,1 or 2.

7. Process according to claim 6, characterized in that the amino acids incorporated in the constitution of the peptide chain of the muramylpeptide which forms part of the immuno-complex are (with the exception of glycine) laevorotatory.

8. Process according to claim 6 or 7, characterized in that the carrier molecule associated with the muramylpeptide which forms part of the immuno-complex is constituted by an antigen, by a molecule having immunogenic properties or by a hapten carrying an antigenic determinant characteristic of this antigen or of this immunogenic molecule.

9. Process for the preparation of a composition according to any of claims 1 to 8 which besides is capable of causing stimulation of the immune response in vivo against a specific antigen, characterized in that with this composition one also associates an immunogenic agent capable of inducing the production in vivo of antibodies active against the said antigen.

10. Process according to any of claims 1 to 9, characterized in that one associates the said muramylpeptide or oligo-muramylpeptide with the antibody in relative proportions of the muramylpeptide and the antibody of the order of 1 molecule of muramylpeptide per 10 to 100 molecules of antibody.

11. Process according to any of claims 1 to 10, characterized by the fact that one dispenses the composition in the form of unit doses of from 1 to 100 micrograms/kg of muramylpeptide equivalent.

FIG.1.

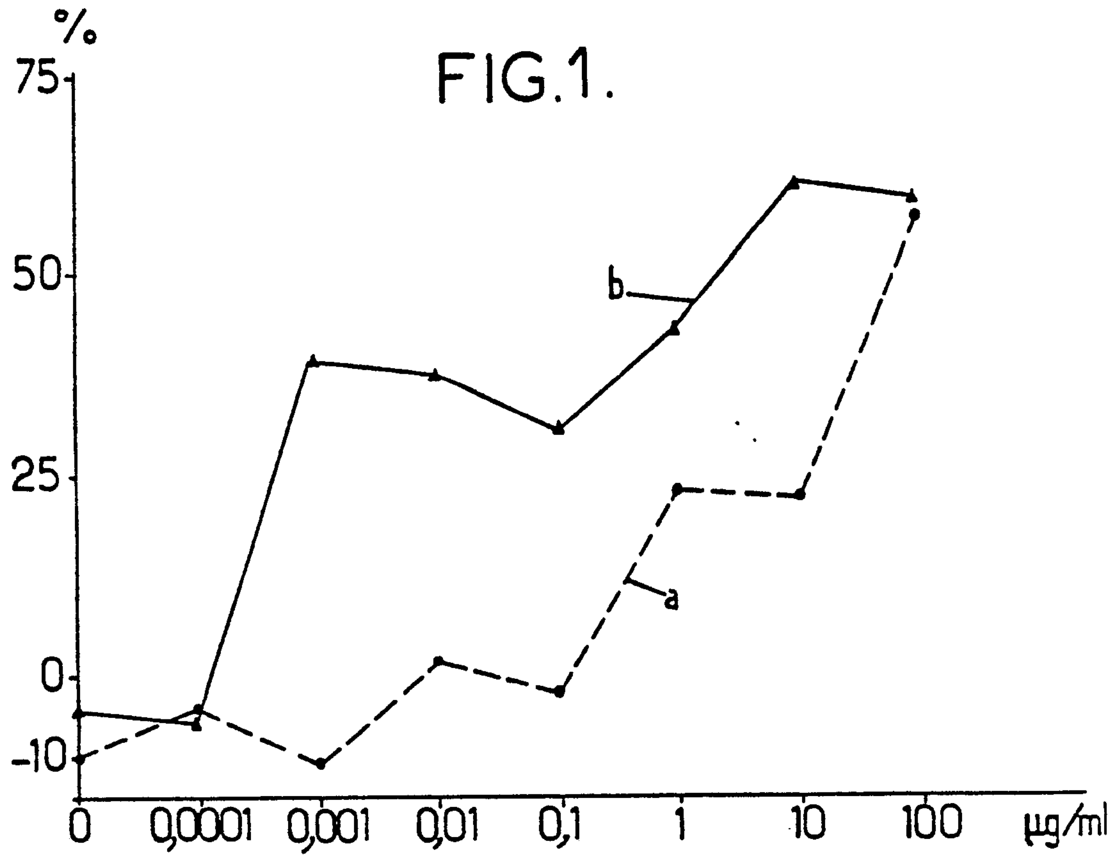
%
75
50
25
0
-10
b
a
0
0,0001
0,001
0,01
0,1
1
10
100
µg/ml

FIG.2.

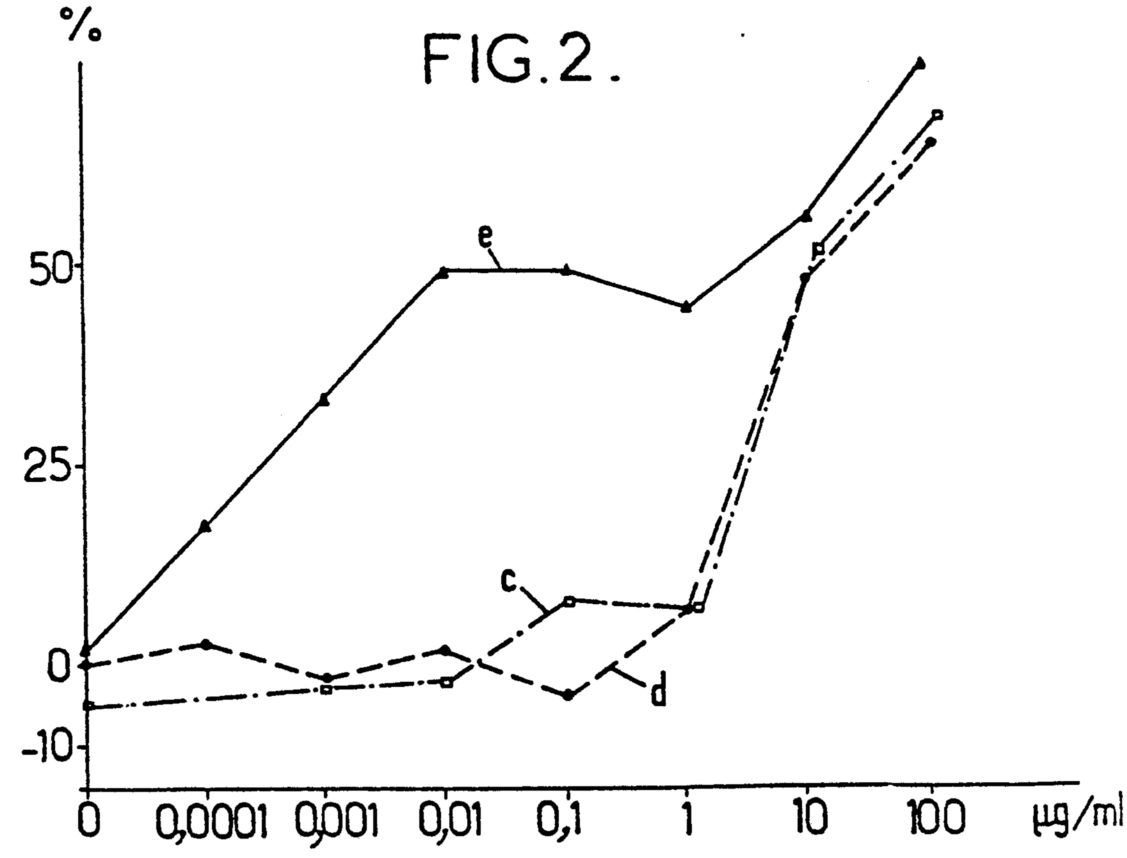
%
50
25
0
-10
e
c
d
0
0,0001
0,001
0,01
0,1
1
10
100
µg/ml

FIG.3.

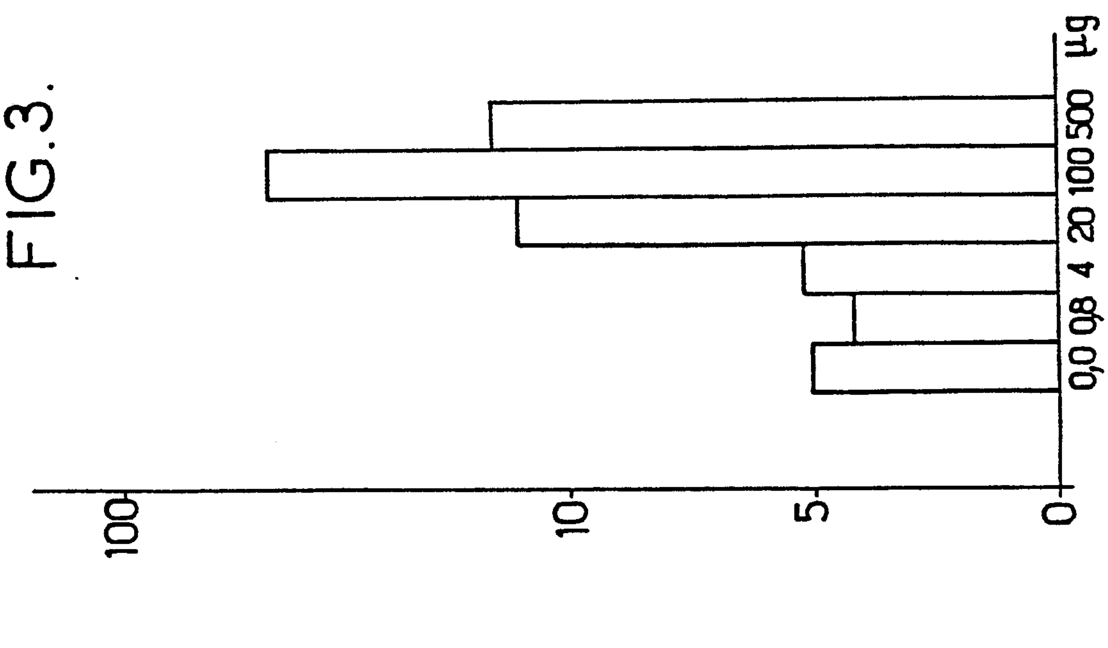


FIG.4.